(19) Europäisches Patentamt
European Patent Office
Office européen des brevets

(11) **EP 4 179 104 B1**

(12) **EUROPEAN PATENT SPECIFICATION**

(45) Date of publication and mention
of the grant of the patent:
**03.12.2025   Bulletin 2025/49**

(21) Application number: **21739650.6**

(22) Date of filing: **05.07.2021**

(51) International Patent Classification (IPC):
**C12Q 1/00** *(2006.01)*        **G01N 27/30** *(2006.01)*
**A61B 5/145** *(2006.01)*       **A61B 5/1486** *(2006.01)*

(52) Cooperative Patent Classification (CPC):
**A61B 5/14865; A61B 5/14532; C12Q 1/002;
C12Q 1/006; G01N 27/31;** A61B 2562/125

(86) International application number:
**PCT/EP2021/068429**

(87) International publication number:
**WO 2022/008394 (13.01.2022 Gazette 2022/02)**

(54) **ANALYTE SENSOR AND ITS MANUFACTURING**

ANALYTSENSOR UND DESSEN HERSTELLUNG

CAPTEUR D'ANALYTES ET SA FABRICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.07.2020   EP 20184466**

(43) Date of publication of application:
**17.05.2023   Bulletin 2023/20**

(73) Proprietors:
• **F. Hoffmann-La Roche AG**
**4070 Basel (CH)**
Designated Contracting States:
**AL CH CY CZ ES GB GR HR HU IE IS LI MC MK
NO PL RS SK SM TR**
• **Roche Diabetes Care GmbH**
**68305 Mannheim (DE)**
Designated Contracting States:
**AT BE BG DE DK EE FI FR IT LT LU LV MT NL PT
RO SE SI**

(72) Inventors:
• **STECK, Alexander**
**68305 Mannheim (DE)**
• **SLIOZBERG, Kyrylo**
**68305 Mannheim (DE)**

(74) Representative: **Stößel, Matthias**
**Altmann Stößel Dick Patentanwälte PartG mbB
Theodor-Heuss-Anlage 2
68165 Mannheim (DE)**

(56) References cited:
WO-A1-2019/222499        US-A1- 2008 135 408
US-A1- 2009 298 104      US-A1- 2010 094 112
US-A1- 2011 021 889      US-A1- 2013 040 404
US-A1- 2015 005 605

• GERNET S ET AL: "Fabrication and
characterization of a planar electrochemical cell
and its application as a glucose sensor",
SENSORS AND ACTUATORS, ELSEVIER,
SWITZERLAND, vol. 18, no. 1, 1 June 1989
(1989-06-01), pages 59 - 70, XP026565755, ISSN:
0250-6874, [retrieved on 19890601], DOI: 10.1016/
0250-6874(89)87025-8

**Description**

Technical Field

The present invention relates to an analyte sensor comprising a substrate, at least one working electrode, at least one second electrode and a membrane, wherein the membrane is located on top of the at least one second electrode. The present invention further relates to a process for manufacturing the inventive analyte sensor as well as to an analyte sensor system comprising an analyte sensor according to the present invention and an electronics unit. The analyte sensor according to the present invention may mainly be used for conducting an analyte measurement in a body fluid of a user.

Background art

Biosensors for measuring analytes in biological fluids, in particular a sensor which is designed for implantation or subcutaneous insertion to measure body fluids, have to fulfill a variety of functions: on the one hand, the sensor must provide for specific and sensitive measurement without interference from e.g. particular components of body fluids. For this purpose, biosensors are frequently covered with membranes excluding particular compounds in order to allow access to the actual sensing sites only for low molecular weight compounds. While the specificity of biosensors is achieved by using of biorecognition elements, such as enzymes, the sensitivity is often tailored by using of diffusion limiting membranes. Finally, the implanted sensor must be biocompatible, thus there is no inflammation reaction of the body caused, for this purpose, an additional biocompatibility membrane may be applied.

Moreover, with implanted sensors, it is preferred to have sensors which can remain in place for a long period without deterioration of the measurement, in order to spare the patient frequently exchanging the sensor.

Implanted sensors, for example, comprise electrode systems which facilitate measurements of physiologically significant analytes such as, for example, like that of glucose in the patient's body. The working electrodes of such a sensor have electrically conductive enzyme layers in which enzyme molecules are bound which release charge carriers by catalytic conversion of the analyte molecules. In this process, an electrical current is generated as measuring signal whose amplitude correlates to the analyte concentration. These types of sensors are also called electrochemical sensors.

US9895091B2 discloses electrochemical sensors. These electrochemical sensors can comprise an impermeable dielectric layer on top of Ag/AgCl of a reference electrode. This coating is used to extend the reference electrode's lifetime. The electrochemical sensor disclosed has a layered structure wherein the reference electrode is positioned on top of a working electrode. The working electrode is separated from the reference electrode by an insulating layer.

US10470691B2 discloses an analyte sensor which comprises a working electrode and a reference electrode. The sensor may comprise an insulator formed of an insulating material. A portion of the insulator may be removed to expose the working electrode and/or the reference electrode.

The manufacturing of the sensors disclosed in the prior art is very time- and cost-consuming. Further, they have drawbacks with regard to their long-term stability.

Problem to be solved

It is therefore an object of the present invention to provide an analyte sensor which avoids at least in part certain drawbacks of the prior art, in particular with regard to its manufacturability and also with regard to its long-term stability.

Summary of the invention

This problem is solved by an analyte sensor according to independent claims 1 as well as by the method for manufacturing this sensor according to independent claim 11 and by the analyte sensor system according to independent claim 13. Preferred embodiments of the invention which may be realized in an isolated way or in any arbitrary combination are disclosed in the dependent claims and throughout the specification

The inventive analyte sensor is particularly easy to manufacture. It furthermore exhibits an excellent long-term stability and a stable sensitivity. In particular, the inventive analyte sensor allows the analyte sensor to comprise only two electrodes instead of three, which makes the inventive analyte sensor particularly cost-efficient. Further, it has an improved biocompatibility

As used in the following, the terms "have", "comprise", or "include" or any arbitrary grammatical variations thereof are used in an exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i. e. a situation in which A solely and exclusively consists of B) and to a situation in which, besides B, one or more further elements are present in entity A, such as element C, elements C and D or even further elements.

Further, it should be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, notwithstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restrictions regarding alternative embodiments of the invention, without any restrictions regarding the scope of the invention and without any restrictions regarding the possibility of combining the features introduced in such way with the optional or nonfunctional features of the invention.

In a first aspect of the present invention an analyte sensor comprising

- a substrate comprising a first side and a second side;
- at least one working electrode positioned on the first side of the substrate, the at least one working electrode comprising:

    - at least one electrically conductive material and
    - at least one enzyme;

- at least one second electrode positioned on the second side of the substrate, the at least one second electrode comprising silver;
- a membrane comprising a polymer composition which comprises a hydrophobic polymer, wherein the hydrophobic polymer has a water uptake of less than 1 % by weight, based on the total weight of the hydrophobic polymer, wherein the membrane is located on top of the at least one second electrode, wherein the membrane comprises holes, wherein the total area of holes has a size of at most 0.15 mm$^2$.

The term "analyte sensor" within the context of the present invention may refer to any device being configured for the detection of an analyte.

The term "analyte" may refer to any arbitrary element, component or compound which may be present in a body fluid and the concentration of which may be of interest for the user. Preferably, the analyte may be or may comprise an arbitrary chemical substance or chemical compound which may take part in the metabolism of the user, such as at least one metabolite. As an example, the analyte may be selected from the group consisting of glucose, cholesterol, triglycerides, and lactate. Additionally or alternatively, however, other types of analytes and/or any combination of analytes may be determined. Preferably, the analyte is glucose.

Thus, the analyte sensor is preferably a biosensor. Further preferably, the analyte sensor is an electrochemical sensor. The term "electrochemical sensor" refers to a sensor which is adapted for performing at least one electrochemical measurement, in particular, a plurality or series of electrochemical measurements, in order to detect the analyte comprised within the body fluid by using an amperometric method. Especially, the term "electrochemical measurement" refers to the detection of an electrochemically detectable property of the analyte, such as an electrochemical detection reaction, by employing amperometric methods. Thus, for example, the electrochemical detection may be carried out by applying and comparing one or more electrical potentials. Specifically, the electrochemical sensor may be adapted to generate at least one electrical measurement signal which may directly or indirectly indicate the presence and/or absence of the electrochemical detection reaction, such as at least one current signal and/or at least one voltage signal. The measurement may be a quantitative and/or a qualitative measurement.

In a particularly preferred embodiment of the present invention, the analyte sensor may be fully or partially implantable and may, thus, be adapted for performing the detection of the analyte in the body fluid in the subcutaneous tissue, in particular, an interstitial fluid. As used herein the terms "implantable" or "subcutaneous" refer to be fully or at least partially arranged within the body tissue of the user, preferably partially arranged within the body tissue of the user. For this purpose, the analyte sensor may comprise an insertable portion, wherein the term "insertable portion" may generally refer to a part or component of an element configured to be insertable into an arbitrary body tissue while other parts or components may remain outside of the body tissue. Preferably, the insertable portion may fully or partially comprise a biocompatible membrane, i. e. a surface which may have as little detrimental effects on the user, the patient, or the body tissue as possible, at least during typical durations of use.

Thus, preferably, the analyte sensor of the present invention is an implantable sensor.

As generally used, the term "body fluid" may refer to fluid, in particular liquid, which may typically be present in a body or a body tissue of the user or the patient and/or which may be produced by the body of the user or the patient. Preferably, the body fluid may be selected from the group consisting of blood and interstitial fluid. However, additionally or alternatively, one or more other types of body fluid may be used, such as saliva, tear fluid, urine or other body fluids. During the detection of the analyte, the body fluid may be present within the body or body tissue. Thus, the analyte sensor may be configured for detecting the analyte within the body tissue. The analyte sensor is in one embodiment suitable for short-term application, e. g. 3 to 21 days, or for long-term application e. g. 1 to 12 months. During its application, the analyte may be determined by continuous or discontinuous measurements.

The analyte sensor of the invention is an electrochemical sensor comprising at least one working electrode and at least one second electrode. More particularly, the sensor is an amperometric electrochemical sensor comprising the at least one working electrode and the at least one second electrode. The working electrode is sensitive for the analyte to be measured at a polarization voltage which may be applied between the at least one working electrode and the at least one second electrode and which may be regulated by a potentiostat. A measurement signal may be provided as an electric current between the at least one working electrode and the at least one second electrode.

The inventive analyte sensor comprises a substrate which comprises a first side and a second side.

Within the context of the present invention, the term "substrate" specifically may refer, without limitation, to any kind of material or combination of materials which is suitable to form a carrier layer to support the at least one working electrode and the at least one second electrode. In particular the substrate may comprise an electrically insulating material. Within the context of the present invention "electrically insulating material" is a broad term and given its ordinary and customary meaning to a person of ordinary skill in the art. The term "electrically insulating material" may also encompass a dielectric material. The term specifically may refer, without limitation, to a material or combination of materials which prevent the transfer of electrical charges and which do not sustain a significant electrical current. Specifically, without limiting other possibilities, the at least one electrically insulating material may be or may comprise at least one insulating resin such as insulating epoxy resins used in manufacturing of electronic printed circuit boards. In particular, it may comprise or be at least one thermoplastic material such as a polycarbonate, a polyester, a polyvinylchloride, a polyurethane, a polyethylene, a polypropylene, polystyrene, a polyether, a polyamide, a polyimide, polytetrafluoroethylene or a copolymer thereof.

In an embodiment, the at least one electrically insulating material may comprise or may be alumina. Suitable polyesters are, for example, selected from the group consisting of polyethylene terephthalate (PET), glycol modified polyethylene terephthalate, and polyethylene naphthalate. A suitable polyethylene is for example selected from the group consisting of high density polyethylene (HDPE) and low density polyethylene (LDPE).

Thus in a preferred embodiment the substrate comprises at least one electrically insulating material selected from the group consisting of an insulating epoxy resin, a polycarbonate, a polyester, a polyvinylchloride, a polyurethane, a polyethylene, a polypropylene, polystyrene, a polyether, a polyamide, a polyimide, polytetrafluoroethylene or a copolymer thereof, and alumina.

The substrate comprises a first side and a second side. To the person skilled in the art it is clear that the first side and the second side are different from one another.

In an embodiment the first side and the second side are positioned opposite each other. Therefore, in an embodiment the substrate comprises two opposing sides, the first side and the second side opposing the first side.

Thus it is preferred that in the analyte sensor according to the present invention the first side and the second side of the substrate are positioned opposite each other.

The substrate may be flat substrate. Specifically the substrate may be flexible and/or deformable. In particular, the substrate may be bendable. Thus, as an example, the substrate may be a thin, flexible substrate. As an example, the substrate may have a thickness of 50 $\mu$m to 1 mm, specifically a thickness of 80 $\mu$m to 500 $\mu$m, such as 110 $\mu$m to 250 $\mu$m.

The substrate may have a length which is preferably less than 50 mm, such as a length of 30 mm or less, e.g. a length of 5 mm to 30 mm.

If the analyte sensor is an implantable sensor, preferably a partially implantable sensor, then the length of the substrate is measured in the insertion direction of the analyte sensor. The length of the substrate refers to the total length of the substrate. The "total length of the substrate" is the overall length of the substrate, including the insertable portion of the substrate which is within the body tissue of the user during use of the analyte sensor and the on body portion of the substrate. The "on body portion of the substrate" is the portion of the substrate which may, for example be connected to the electronics unit.

The analyte sensor comprises at least one working electrode positioned on the first side of the substrate. Preferably the at least one working electrode is positioned only on the first side of the substrate. This means, within the context of the present invention, that in an embodiment the second side does not comprise at least one working electrode.

The at least one working electrode is preferably adapted for detecting the analyte, in particular, the at least one working electrode is an electrode of the analyte sensor which is sensitive for the analyte.

The at least one working electrode comprises at least one electrically conductive material. "Electrically conductive material" within the context of the present invention refers to a material being capable of sustaining an electrical current. Thus, the at least one electrically conductive material may be selected from the group consisting of metals, and nonmetallic electrically conductive materials.

Suitable metals are known as such and are, for example, selected from the group consisting of gold, nickel, platinum, and palladium, wherein gold is particularly preferred.

Suitable nonmetallic electrically conductive materials are for example selected from the group consisting of carbon, carbon paste, gold paste or conductive polymers. Suitable conductive polymers are, for example polyaniline and/or poly-3,4-ethylenedioxythiophene (PEDOT). Carbon paste may comprise, for example, carbon and a solvent such as diethylene glycol butyl ether and at least one binder such as vinyl chloride co- and terpolymers. Carbon paste is known as such.

Thus, the at least one electrically conductive material of the at least one working electrode preferably is selected from the group consisting of gold, nickel, platinum, palladium, carbon, carbon paste, polyaniline and poly-3,4-ethylenedioxythiophene (PEDOT), particularly preferred, the at least one electrically conductive material of the at least one working electrode is selected from the group consisting of gold, carbon, and carbon paste. More preferably, the at least one electrically conductive material consists essentially of gold and/or carbon and/or carbon paste. In an embodiment, the at least one electrically conductive material has a layered structure wherein a first layer consists of gold and a second layer consists of carbon and/or carbon paste. In this embodiment, preferably, gold is positioned on top of the first side of the substrate and on top of the gold, carbon and/or carbon paste is positioned.

In particular, the at least one working electrode may comprise the at least one electrically conductive material in the form of at least one conductive trace. The term "conductive trace" within the context of the present invention refers, without limitations, to an electrically conductive strip, layer, wire or other type of electrical conductor. The conductive trace may have a thickness of at least 0.05 $\mu$m, preferably of at least 0.5 $\mu$m, more preferably of at least 5 $\mu$m, specifically of at least 7 $\mu$m, or at least 10 $\mu$m. In the case where the conductive trace comprises carbon or is carbon, the conductive trace may specifically have a thickness of at least 7 $\mu$m, more specifically of at least 10 $\mu$m. Specifically, in the case where the conductive trace is gold, the conductive trace may have a thickness of at least 50 nm, more specifically of at least 900 nm.

The at least one electrically conductive material may be positioned on the first side of the substrate by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

The at least one working electrode comprises at least one enzyme. The at least one working electrode may comprise precisely one enzyme or a mixture of two or more enzymes. Precisely one enzyme is preferred. Specifically, the enzyme is capable of catalyzing a chemical reaction converting the analyte. Even more specifically the at least one enzyme is selected from the group consisting of a glucose oxidase (EC 1.1.3.4), a hexose oxidase (EC 1.1.3.5), an (S)-2 hydroxy acid oxidase (EC 1.1.3.15), a cholesterol oxidase (EC 1.1.3.6), a glucose dehydrogenase, a galactose oxidase (EC 1.1.3.9), an alcohol oxidase (EC 1.1.3.13), an L-glutamate oxidase (EC 1.4.3.11), and an L-aspartate oxidase (EC 1.4.3.16). In particular, the at least one enzyme is a glucose oxidase (GOx) and/or modifications thereof.

The at least one enzyme may be comprised in a sensing material. The sensing material which comprises the at least one enzyme may be located at least partially on the electrically conductive material of the at least one working electrode. In particular, the sensing material may cover at least a portion of the at least one conductive trace. The sensing material in conjunction with the conductive trace forms the at least one working electrode. In particular, the sensing material preferably forms a layer on the at least one electrically conductive material.

The sensing material may be applied by any known method to the at least one electrically conductive material, for example by a wet-coating process. A suitable wet-coating process is for example selected from the group consisting of spin-coating, spray-coating, doctor-blading, printing, dispensing, slot-coating, dip coating and screen printing. After the wet-coating process, the layer of the sensing material may be further treated. Such treatments are for example drying treatment, curing treatments and/or laser ablation treatments. Such treatments are known as such.

The term "sensing material", as used herein, is a broad term and is to be given its ordinary and customary meaning to a person of ordinary skill in the art and is not to be limited to a special or customized meaning. The term specifically may refer, without limitation, to a material that may be or may comprise at least a polymeric material; specifically it may be or may comprise at least a polymeric material and at least a metal containing complex. The metal containing complex may be selected from the group of transition metal element complexes, specifically the metal containing complex may be selected from osmium-complexes, ruthenium-complexes, vanadium-complexes, cobalt-complexes, and iron-complexes, such as ferrocenes, such as 2-aminoethylferrocene. Even more specifically, the sensing material may be a polymeric transition metal complex as described for example in WO 01/36660 A2, the content of which is included by reference. In particular, the sensing material may comprise a modified poly(vinylpyridine) backbone loaded with poly(biimidizyl) Os complexes covalently coupled through a bidentate linkage. A suitable sensing material is further described in Feldmann et al, Diabetes Technology & Therapeutics, 5 (5), 2003, 769-779, the content of which is included by reference. Suitable sensing materials further may include ferrocene-containing polyacrylamide-based viologen-modified redox polymer, pyrrole-2,2'-azino-bis(3-ethylbenzthiazoline-6-sulfonic acid)(ABTS)-pyrene, Naphthoquinone-LPEI. The polymeric transition metal complex may represent a redox mediator incorporated into a cross-linked redox polymer network. This is advantageous as it may facilitate electron transfer between the at least one enzyme or analyte and the conductive trace. In order to avoid a sensor drift, the redox mediator and the enzyme may be covalently incorporated into a polymeric structure.

In an embodiment the sensing material may comprise a polymeric material and $MnO_2$-particles or any other material catalyzing hydrogen peroxide oxidation reaction as well as the at least one enzyme. Another material catalyzing hydrogen peroxide oxidation reaction is Pt (platinum).

Moreover, the sensing material may additionally comprise at least one crosslinker; the crosslinker may for example be capable of crosslinking at least part of the sensing material. Specifically the sensing material may comprise at least one crosslinker selected from UV-curable crosslinkers and chemical crosslinkers; more specifically the sensing material comprises a chemical crosslinker. Alternatively, the sensing material may be free of any crosslinker. "Free of any crosslinker" as used herein, is a broad term and is to be given its ordinary and customary meaning to a person of ordinary skill in the art and is not to be limited to a special or customized meaning. The term specifically may refer to a concentration of crosslinker in the range from 0 to 0.5 wt-% based on the dry weight of the sensing material. The term "dry weight" as used herein refers to the dry matter of the respective material, e.g. the material without the addition of any water or other solvent.

Suitable chemical crosslinkers according to the present invention are preferably selected from the group consisting of epoxide based crosslinkers, such as diglycidyl ethers like poly(ethylene glycol) di-glycidyl ether (PEG-DGE) and poly(propylene glycol) diglycidyl ether; trifunctional short chain epoxides; anhydrides; diglycidyl ethers such as Resorcinol diglycidyl ether, Bi-sphenol A diglycidyl ether, Diglycidyl 1,2-cyclohexanedicarboxylate, Poly(ethylene glycol) diglycidyl ether, Glycerol diglycidyl ether, 1,4-Butanediol diglycidyl ether, Poly(propylene glycol) diglycidyl ether, Bisphenol diglycidyl ether, Poly(dimethylsiloxane), diglycidyl ether, Neopentyl glycol diglycidyl ether, 1,2,7,8-Diepoxyoctane, 1,3-Glycidoxypropyl-1,1,3,3-Tetramethyldisioxane; triglycidyl ethers such as N,N-Diglycidyl-4-glycidyloxyaniline, Trimethylolpropane triglycidyl ether; and tetraglycidyl ethers such as Tetrakisepoxy cyclosiloxane, Pentaerythritol tetraglycidyl ether, tetraglycidyl-4,4'-methylenebisbenzenamine.

The term "chemical crosslinker" as used herein, is a broad term and is to be given its ordinary and customary meaning to a person of ordinary skill in the art and is not to be limited to a special or customized meaning. The term specifically may refer, without limitation, to a crosslinker that is capable of initiating a chemical reaction generating a crosslinked molecular network and/ or a cross-linked polymer when exposed to heat. "Exposed to heat" may refer to being exposed to a temperature above 15°C, specifically to a temperature above 20 °C; more specifically to a temperature in the range from 20 °C to 50 °C and even more specifically to a temperature in the range from 20 °C to 25 °C. More specifically, chemical crosslinkers may initiate crosslinking of the sensing material when exposed to heat.

The term "UV-curable crosslinker" as used herein, is a broad term and is to be given its ordinary and customary meaning to a person of ordinary skill in the art and is not to be limited to a special or customized meaning. The term specifically may refer, without limitation, to the ability of a chemical substance of initiating a photochemical reaction generating a crosslinked molecular network and/ or a crosslinked polymer when irradiated by light in the UV spectral range. More specifically, UV-curable crosslinkers may initiate crosslinking of the layer of the sensing material when irradiated by UV light.

Suitable UV curable crosslinkers according to the present invention include: benzophenone, diazirine and azide. Particularly suitable UV-curable crosslinkers are for example selected from the group consisting of, benzophenone comprising cross-linkers, poly(Di(2-hydroxy 3 aminobenzophenonepropylene) glycol), Dibenzophenone 1,2-cyclohexanedicarboxylate, Bis[2-(4-azidosalicylamido)ethyl] Disulfide, reaction products of the reaction of 4-aminobenzophenone with any one of the above for the chemical cross-linker described di-glycidyl cross-linkers, triglycidyl cross-linkers and tetraglycidyl cross-linkers, an example of such a reaction product is 2,4,6,8-Tetramethyl-2,4,6,8-tetrakis(2-hydroxy 3-aminpropylbenzophenone)-cyclotetrasiloxan, and reaction products of the reaction of 4-Benzoylbenzoic Acid N-Succinimidyl Ester with a diamin or a jeffamin.

The analyte sensor furthermore comprises at least one second electrode positioned on the second side of the substrate. Preferably, the at least one second electrode is positioned only on the second side of the substrate. This means, within the context of the present invention, that in an embodiment, the first side does not comprise at least one second electrode. In an embodiment, precisely one second electrode is positioned on the second side of the substrate. Preferably, the at least one second electrode does not comprise an enzyme. Thus, preferably, the at least one second electrode is free of the at least one enzyme. Preferably, the second side of the substrate is free of enzyme.

The at least one second electrode may be selected from the group consisting of a counter electrode, a reference electrode, and a combined counter/reference electrode. Preferably, the at least one second electrode is a combined counter/reference electrode.

Thus, it is preferred that the at least one second electrode of the analyte sensor is selected from the group consisting of a counter electrode, a reference electrode and a combined counter/reference electrode.

The at least one second electrode comprises silver. "Silver" within the context of the present invention does not only encompass elemental silver, but also any silver containing compound. Therefore, the at least one second electrode comprises elemental silver and/or at least one silver containing compound. A preferred silver containing compound is silver chloride (AgCl). For example, the at least one second electrode comprises elemental silver and/or silver chloride. In particular, the at least one second electrode comprises elemental silver and silver chloride. In particular, the at least one second electrode comprises silver / silver chloride (Ag/AgCl). In an embodiment, the at least one second electrode only comprises AgCl when the analyte sensor is manufactured. No elemental Ag is added when the analyte sensor is manufactured. During use of the analyte sensor, elemental Ag may then be formed from AgCl, so that during use, the analyte sensor comprises Ag/AgCl. The reaction of AgCl to form elemental Ag is known to the skilled person as such.

Thus, an analyte sensor which comprises at least one second electrode which comprises Ag/AgCl is preferred.

For example, the load of AgCl of the at least one second electrode is in the range from 20 $\mu$g to 150 $\mu$g. If two or more second electrodes are comprised, then the load of AgCl of the at least one second electrode refers to the sum of the load of AgCl of the two or more second electrodes. The load of AgCl of the at least one second electrode refers to the load when the analyte sensor is manufactured. It is clear to the skilled person that the load may change during use of the analyte sensor, for example due to the formation of elemental Ag from AgCl.

Thus, an analyte sensor in which the load of AgCl of the at least one second electrode is in the range from 20 $\mu$g to 150 $\mu$g is preferred.

The minimum load of AgCl of the at least one second electrode may be calculated according to the following formula.

$$m(AgCl) = \frac{M(AgCl) * I * t}{z * F}$$

wherein

I      is the highest possible current in A while the analyte sensor is in use
t      is the total wear time of the sensor in s
F      is the Faraday constant in C/mol
z      is the charge number of silver (z = 1)
M(AgCl)      is the molar mass of AgCl
m(AgCl)      is the load of AgCl of the at least one second electrode.

Ag/AgCl which the at least one second electrode in an embodiment comprises may be comprised in a binder. Suitable binders are known as such and are, for example, selected from the group consisting of metallic binders, ceramic binders and polymeric binders. Preferred are polymeric binders, in particular physically binding polymer binders and/or chemically binding polymer binders.

For example, Ag/AgCl which the at least one second electrode comprises, comprises in the range from 50 to 70 wt.-% of Ag, in the range from 20 to 40 wt.-% of AgCl and in the range of 1 to 20 wt.-% of a binder, wherein the wt.-% are in each case based on the sum of the wt.-% of Ag, AgCl and the binder.

The at least one second electrode may comprise at least one second conductive trace. The at least one second conductive trace is preferably positioned on the second side of the substrate. In particular, the first side of the substrate preferably does not comprise a second conductive trace.

The term "second conductive trace" specifically may refer, without limitation, to an electrically conductive strip, layer, wire or other type of elongated electrical conductor. In particular, the term may refer to at least one second electrically conductive material. Hence, the at least one second conductive trace is preferably capable of sustaining an electrical current. For example. the at least one second electrically conductive material may be selected from the group consisting of gold, nickel, platinum, palladium, carbon, carbon paste, polyaniline and poly-3,4-ethylenedioxythiophene (PEDOT). Particularly preferred, the at least one second electrically conductive material of the at least one second electrode is selected from the group consisting of gold, carbon, and carbon paste. More preferably, the at least one second electrically conductive material consists essentially of gold and/or carbon and/or carbon paste. In an embodiment, the at least one second electrically conductive material has a layered structure wherein a first layer consists of gold and a second layer consists of carbon and/or carbon paste. In this embodiment, preferably, gold is positioned on top of the second side of the substrate and on top of the gold, carbon and/or carbon paste is positioned.

It is preferred that silver which the at least one second electrode comprises is positioned on top of the at least one second conductive trace. Thus, it is preferred, that Ag/AgCl is positioned at least partially on top of the at least one second conductive trace, in particular on top of the layered structure of the at least one second electrically conductive material. Silver, preferably Ag/AgCl, and the at least one second conductive trace, in particular the layered structure of the at least one second conductive material, form the at least one second electrode.

Thus, the silver which the at least one second electrode comprises typically forms a layer. The thickness of the layer is for example in the range from 5 $\mu$m to 30 $\mu$m.

The at least one second conductive trace and the at least one second electrically conductive material may be applied to the second side of the substrate by the same methods as the methods by which the at least one conductive trace and the at least one electrically conductive material of the at least one working electrode are applied to the first side of the substrate. Thus, the embodiments and preferences described above hold true. The methods for applying the at least one second conductive trace and the at least one second electrically conductive material of the second electrode and the methods for applying the at least one conductive trace and the at least one electrically conductive material of the working electrode may be selected independent from one another.

Silver, in particular Ag/AgCl, comprised in the at least one second electrode may be applied to the second side of the substrate, in particular at least partially on top of the at least one second conductive trace, by the same methods as the methods by which the sensing material of the at least one working electrode is applied to the first side of the substrate. Thus, the embodiments and preferences described above hold true. The method for applying silver, in particular Ag/AgCl, comprised in the at least one second electrode and the method for applying the sensing material comprised preferably in the at least one working electrode may be selected independent from one another.

The analyte sensor of the present invention comprises a membrane. The membrane comprises a polymer composition which comprises a hydrophobic polymer and is located on top of the at least one second electrode.

In an embodiment, the term "membrane" within the context of the present invention refers to a layer of at least one material which provides a selective barrier. Thus, such a membrane generally may selectively allow for one or more molecules and/or compounds to pass through the membrane, whereas other molecules and/or compounds are stopped by the membrane. For example, the membrane allows transfer of water and/or chloride anions through the membrane and restricts transfer of silver cations and/or silver chloride through the membrane.

For example, in this embodiment, the membrane may have a thickness in the range from 1 $\mu$m to 100 $\mu$m, preferably in the range from 5 $\mu$m to 20 $\mu$m.

The term "membrane" within the context of the present invention refers to a layer of at least one material which is essentially impermeable. "Essentially impermeable" means that the membrane has a water uptake of less than 1 % by weight, based on the total weight of the membrane.

For example, the membrane may have a thickness in the range from 1 $\mu$m to 100 $\mu$m, preferably in the range from 5 $\mu$m to 15 $\mu$m.

The embodiments described in the following with regard to the membrane apply to both embodiments of the membrane, preferably to embodiment of the membrane referring to a layer of at least one material which is essentially impermeable.

The membrane is located on top of the at least one second electrode.

"Located on top of the at least one second electrode" means that the membrane covers the at least one second electrode. In an embodiment, the membrane covers the at least one second electrode fully. The term "cover fully" within the context of the present invention means that the at least one second electrode, when the analyte sensor is implanted into the user, does not have direct contact with the body fluid of the patient. Instead, only the membrane has direct contact with the body fluid.

In a further embodiment, the membrane covers the at least one second electrode partially. The term "covers partially" within the context of the present invention means that the at least one second electrode, when the analyte sensor is implanted into the user, does have direct contact with the body fluid. In particular, the at least one second electrode has direct contact with the body fluid via holes in the membrane. This embodiment is particularly preferred if the term "membrane" refers to a layer of at least one material which is essentially impermeable.

The membrane comprises holes. The membrane may comprise precisely two or more holes.

The term "holes" within the context of the present invention is given its ordinary and customary meaning to a person skilled in the art. In particular, it means any opening and/or perforation within the membrane. The holes allow passage of one or more molecules and/or compounds through the holes. Thus, the holes provide a fluidic channel between the at least one second electrode and the body fluid.

The holes may have any shape and any size. The holes may be positioned anywhere within the membrane. For example, the holes may be positioned at an edge of the membrane and/or essentially in the center of the membrane.

The membrane comprises holes, wherein the total area of the holes comprised in the membrane is at most 0.15 square millimeters (mm2), preferably at most 0.05 mm2. In an embodiment, the total area of holes comprised in the membrane is in the range from 0.005 to 0.15 mm2, preferably in the range from 0.005 to 0.05 mm2.

Within the context on the present invention, "the total area of holes" relates to the sum of the surface area of the holes.

In a preferred embodiment of the present invention, the at least one working electrode does not comprise the membrane comprising the polymer composition which comprises the hydrophobic polymer.

Thus, an analyte sensor is preferred in which the at least one working electrode is free of the membrane comprising the polymer composition which comprises the hydrophobic polymer.

The membrane comprises a polymer composition which comprises a hydrophobic polymer. "Hydrophobic" within the context of the present invention means that the polymer has a water uptake of less than 1 % by weight, based on the total weight of the polymer.

The hydrophobic polymer is preferably a thermoplastic hydrophobic polymer.

The hydrophobic polymer, for example, has a glass transition temperature in the range from - 100 °C to 0 °C, preferably in the range from -70 °C to -50 °C. The glass transition temperature may be measured via differential scanning calorimetry using a ramp of 10 °C/min for heating and cooling. The glass transition temperature is measured during the second heating cycle. This means that first, the hydrophobic polymer is heated with a ramp of 10 °C/min, then it is cooled with a ramp of 10 °C/min and then it is heated again with a ramp of 10 °C/min to determine the glass transition temperature.

The hydrophobic polymer, for example, has a crystallization temperature in the range from 50 °C to 100 °C, for example in the range from 75 °C to 85 °C. The crystallization temperature is measured via differential scanning calorimetry using the same parameters as for the glass transition temperature.

Thus, an analyte sensor is preferred wherein the hydrophobic polymer has a glass transition temperature, wherein the glass transition temperature is in the range from -100 °C to 0 °C.

This glass transition temperature is particularly advantageous as it results in a sufficiently high stability of the membrane. If the sensor is bent, in particular during use, the membrane will not or only to a small extend be damaged.

The hydrophobic polymer may be any hydrophobic polymer known to the skilled person. Preferably, the hydrophobic polymer is selected from the group consisting of thermoplastic polyurethanes (TPU), thermoplastic polyurea, polyethylene, polypropylene, polystyrene, butyl methacrylate polymers (BUMA), polyethylene terephtalate (PET), and UV hardening resins, such as acrylated silicones, acrylated urethanes, acrylated polyesters and/or acrylated epoxides. Preferably, the hydrophobic polymer is a thermoplastic polyurethane.

Therefore, an analyte sensor is preferred, wherein the polymer composition comprises a hydrophobic thermoplastic polyurethane.

The hydrophobic thermoplastic polyurethane may comprise hard segments and soft segments in various ratios. Suitable hard segments usually comprise a polymerization product of a diisocyanate and a polyol. A suitable diisocyanate may be an aliphatic diisocyanate or an aromatic diisocyanate, preferably an aliphatic diisocyanate.

Suitable aromatic diisocyanates are for example, 4,4'-methylene diphenyl diisocyanate, and/or toluene-2,4-diisocyanate.

Suitable aliphatic diisocyanates are for example, hexamethylene diisocyanate, and/or isophorone diisocyanate.

A suitable polyol is preferably a diol, such as 1,4-butanediol, 1,5-pentanediol, 1,6-hexanediol, and/or 1,10-decanediol.

Suitable soft segments may comprise polyethers and/or polyesters. Suitable polyethers are for example polyethylene oxide and/or polytetrahydrofurane, whereas suitable polyesters are for example polyethylene terephthalate and/or polyethylene naphthalate.

The polymer composition may comprise further components.

The analyte sensor may comprise at least one third electrode. Preferably, the analyte sensor does not comprise at least one third electrode.

If at least one third electrode is comprised in the analyte sensor, then the at least one second electrode is preferably selected from the group consisting of a counter electrode and a reference electrode. The at least one third electrode is then preferably also selected from the group consisting of a counter electrode and a reference electrode. If the at least one second electrode is a counter electrode, then the at least one third electrode is a reference electrode and vice versa.

The analyte sensor may further comprise at least one flux limiting membrane.

The at least one flux limiting membrane is specifically at least positioned on top of the at least one working electrode. Preferably, the at least one flux limiting membrane is also positioned on top of the membrane comprising the polymer composition which comprises the hydrophobic polymer.

The term "flux limiting membrane", as used herein, is a broad term and is to be given its ordinary and customary meaning to a person of ordinary skill in the art and is not to be limited to a special or customized meaning. The term specifically may refer, without limitation, to a layer of at least one material, which provides a selective barrier. Thus, the flux limiting membrane generally may selectively allow for one or more molecules and/or compounds to pass, whereas other molecules and/or compounds are stopped by the flux limiting membrane. Thus, the flux limiting membrane is permeable for the at least one analyte to be detected. Thus, as an example, the flux limiting membrane may be permeable for one or more of glucose, lactate, cholesterol or other types of analytes. The at least one flux limiting membrane may, hence, function as a diffusion barrier that controls diffusion of the analyte from the exterior, e.g. the body fluid surrounding the analyte sensor, to the sensing material, i. e. the at least one enzyme which the at least one working electrode comprises. In addition, the at least one flux limiting membrane may function as a biocompatibility membrane layer as mentioned elsewhere herein.

The at least one flux limiting membrane, as an example, may have a thickness sufficient for providing mechanical stability. The at least one flux limiting membrane specifically may have a thickness of 1 μm to 150 μm. For the at least one flux limiting membrane, as outlined herein, several materials may be used, standalone or in combination. Thus, as an example, the flux limiting membrane specifically may comprise at least one polymeric material. Suitable polymeric materials may, for example be selected from the group consisting of a polyvinylpyridine based copolymer, a polyurethane and a hydrogel. Polyvinylpyridine based copolymers are particulary suitable.

Suitable hydrogels are in particular polyethylene glycol copolymers (PEG copolymers), polyvinyl acetate copolymers (PVA copolymers), poly(2-alkyl-2-oxazonline) copolymers, polyacrylate and/or methacrylate-acrylate copolymers or block-copolymers, in particular polyacrylate and/or methacrylate-acrylate copolymers or block-copolymers comprising hydrophilic side groups. Thus, as an example, suitable hydrogels may be selected from the group consisting of (hydroxyethyl) methacrylate (HEMA) -homopolymers, HEMA-copolymers, silicon hydrogels and HEMA-co-N-vinylpyrrolidone-polymers, each of which may comprise side groups selected from the group consisting of methacrylic acid, glycerol methacrylate, N,N-dimethylacrylamide and phospharylcholine.

These types of flux limiting membranes are generally known in the art. As an example, flux limiting membranes as disclosed in e.g. EP2697388 A1, WO 2007/071562 A1 and/or in WO 2005/078424 A1 may be used. Specifically, the polymeric material may have a weight average molecular weight (MW) of more than 10.000 kDa. More specifically, the polymeric material may have a weight average molecular weight (MW) of more than 50.000 kDa, or even more than 100.000 kDa. Particularly suitable are polymeric materials with a weight average molecular weight (MW) of 10.000 to 500.000 kDa. The polymeric material of the flux limiting membrane may be the same as or may be different from the polymeric material of the sensing material.

The analyte sensor may further comprise at least one biocompatibility membrane.

The at least one biocompatibility membrane is specifically at least positioned on top of the at least one working electrode. Preferably, the at least one biocompatibility membrane is also positioned on top of the membrane comprising the polymer composition which comprises the hydrophobic polymer. In particular, the at least one biocompatibility membrane is positioned on top of the flux limiting membrane which is in an embodiment of the present invention comprised in the analyte sensor. Specifically, the biocompatibility membrane fully covers the at least one flux limiting membrane. The term "fully covers" within the context of the present invention means that, specifically, the flux limiting membrane is not in direct contact with a body fluid if the analyte sensor is in use, but only the biocompatibility membrane is in direct contact with the body fluid. This means that at least the implantable portion of the analyte sensor is preferably fully covered by the at least one biocompatibility membrane.

The term "biocompatibility membrane ", also denoted biocompatibility layer, as used herein, relates to a layer, in particular an outmost layer of the analyte sensor or part thereof, consisting of a biocompatible material. Specifically, the biocompatibility layer has a thickness of from 1 µm to 10 µm, in an embodiment of from 3 µm to 6 µm. More specifically, the biocompatibility layer covers the analyte sensor at least partly or completely. Even more specifically, the biocompatibility layer may be the outmost layer of the analyte sensor. Thus, even more specifically, at least a part of the biocompatibility layer contacts a body fluid of a subject. For example, the biocompatibility layer may be not diffusion-limiting for the analyte as specified elsewhere herein. For example, the biocompatibility layer may be not diffusion-limiting for small molecules having a molecular weight of less than 2.000 Da, in an embodiment less than 1.000 Da. For example, the biocompatibility layer may not comprise an added enzyme. For example, the biocompatibility layer may not comprise an added polypeptide. As will be understood by the skilled person, this does not exclude that enzyme or polypeptide molecules diffuse into the biocompatibility layer from adjacent layers, tissues, or body fluids.

The term "biocompatible material", as used herein relates to a material suitable for use with living tissue or a living system by not being or being to a reduced extent toxic, injurious, or physiologically reactive and/or causing to a reduced extent or not causing immunological rejection. In an embodiment, the biocompatible material is a material not inducing a bodily response, e.g. an inert material or a material comprising chemical compounds preventing bodily responses from occurring in the vicinity of the biocompatibility layer. In another embodiment, the biocompatible material is a material pre-venting cells from attaching to said biocompatibility layer. The biocompatibility membrane may be or may comprise at least one material selected from the group consisting of methacrylate based polymers and copolymers, such as acrylamide-methacrylate based copolymers, biodegradable polysaccharides such as hyaluronic acid (HA), agarose, dextran and chitosan. Further biocompatible materials are disclosed in WO 2019/166394 A1 and include nonbiodegradable synthetic hydrogels such as hydrogels prepared from the copolymerization of 2-hydroxyethyl methacrylate (HEMA), 2-hydroxypropyl methacrylate (HPMA), acrylamide (AAm), acrylic acid (AAc), N-isopropylacrylamide (NIPAm), and methoxyl poly(ethylene glycol) (PEG) monoacrylate (mPEGMA or PEGMA), with cross-linkers, such as N,N'-methylenebis(acrylamide) (MBA), ethylene glycol diacrylate (EGDA) and PEG diacrylate (PEGDA), Pluronic® polymers with a structure of poly(ethylene oxide) (PEO)-poly(propylene oxide) (PPO)-PEO, chemical crosslinking of modified poly(vinyl alcohol) (PVA), Poly (4vinylpyridine), PEG.

Another object of the present invention is a method for manufacturing the inventive analyte sensor, the method comprising the steps:

a) providing a raw substrate which comprises a first side and a second side,
b) preparing a working electrode region on the first side of the raw substrate, the preparing of the working electrode region comprising the steps:

b1) applying an electrically conductive material to the first side of the raw substrate,
b2) applying a sensing material comprising at least one enzyme at least partially on the electrically conductive material,

c) preparing a second electrode region on the second side of the raw substrate, the preparing of the second electrode region comprising the steps:
c1) applying a silver composition on the second side of the raw substrate,
d) applying a polymer composition on top of the second electrode region, to obtain a membrane, wherein the polymer composition comprises a hydrophobic polymer, wherein the hydrophobic polymer has a water uptake of less than 1 % by weight, based on the total weight of the hydrophobic polymer,
e) cutting the raw substrate comprising the working electrode region, the second electrode region and the membrane to obtain the analyte sensor.

Process steps a) to e) may be carried out in the given order. However, it is also possible to carry out the steps in different orders. In particular, the order of steps b) and c) may be different. It is even possible, to first carry out step b1), then step c1) and thereafter step b2), for example. Further process steps are feasible. It is also possible to carry out at least one of process steps a) to e) more than once. For example, step c1) may be carried our more than once, so that more than one layer of the silver composition is obtained.

In step a) of the method for manufacturing the inventive analyte sensor, a raw substrate is provided.

Within the context of the present invention, the term "raw substrate" specifically may refer, without limitation, to any kind of material or combination of materials which is suitable to form a carrier layer to support the at least one working electrode and the at least one second electrode. From the raw substrate, the substrate of the inventive analyte sensor may be manufactured, for example, by cutting the raw substrate. In particular, the raw substrate may comprise an electrically insulating material. For the electrically insulating material, the embodiments and preferences described above for the electrically insulating material of the substrate hold true.

Thus, in a preferred embodiment the raw substrate comprises at least one electrically insulating material selected from the group consisting of an insulating epoxy resin, a polycarbonate, a polyester, a polyvinylchloride, a polyurethane, a polyether, a polyethylene, a polyamide, a polyimide, a polyacrylate, a polymethacrylate, a polytetrafluoroethylene or a copolymer thereof, and alumina.

A suitable polyester is for example polyethyleneterephthalate.

The raw substrate comprises a first side and a second side. To the person skilled in the art it is clear that the first side and the second side are different from one another.

In an embodiment the first side and the second side are positioned opposite each other. Therefore, in an embodiment the raw substrate comprises two opposing sides, the first side and the second side opposing the first side.

The raw substrate may be a flat substrate. Specifically the raw substrate may be flexible and/or deformable. Thus, as an example, the raw substrate may be a thin, flexible raw substrate. As an example, the raw substrate may have a thickness of 50 $\mu$m to 1 mm, specifically a thickness of 80 $\mu$m to 500 $\mu$m, such as 110 $\mu$m to 250 $\mu$m.

The raw substrate may have a length which is preferably in the range from a few centimeters to several meters, such as for example in the range from 10 cm to 100 m.

The raw substrate may have a width which is preferably in the range from 2 centimeters (cm) to 8 cm.

In an embodiment, the raw substrate may comprise an electrically conductive material on at least one of the first and the second side, preferably on the first side and on the second side.

In an embodiment of the present invention, the raw substrate may be suitable to be used in a roll-to-roll process.

The raw substrate may be provided by any method known to the skilled person. For example, the raw substrate may be provided as a roll. This is particularly advantageous as the raw substrate may then be used in a roll-to-roll process.

In an embodiment, the raw substrate is cut into sheets before the working electrode region is prepared. The sheets may have any length, such as, for example, in the range from 100 mm to 300 mm.

In step b) a working electrode region is prepared on the first side of the raw substrate.

The working electrode region specifically comprises all components which form part of the at least one working electrode of the analyte sensor.

In step b1) the electrically conductive material is applied to the first side of the raw substrate. For the electrically conductive material the embodiments and preferences described above hold true.

The electrically conductive material can be applied to the first side of the raw substrate by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

In step b2) a sensing material comprising at least one enzyme is applied at least partially on the electrically conductive material. For the sensing material and the at least one enzyme, the embodiments and preferences described above hold true.

The sensing material may be applied by any known method to the at least one electrically conductive material, for example by a wet-coating process. A suitable wet-coating process is for example selected from the group consisting of spin-coating, spray-coating, doctor-blading, printing, dispensing, slot-coating, dip coating and screen printing. After the wet-coating process, the layer of the sensing material may be further treated. Such treatments are for example drying treatments, curing treatments and/or laser ablation treatments. Such treatments are known as such.

The sensing material may be applied to the electrically conductive material so that it fully or partially covers the electrically conductive material, it may also overlap with the electrically conductive material. The sensing material may be applied to the electrically conductive material in any shape. For example in the shape of one or a plurality of lines, one or a plurality of dots, one or a plurality of strips. It is also possible to remove the sensing material partially from the at least one electrically conductive material after its application. Methods to remove the sensing material partially from the at least one electrically conductive material are known as such. For example, a portion of the sensing material may be irradiated by light, in particular by a laser, thereby removing the sensing material partially. It is also possible to irradiate a portion of the sensing material, thereby crosslinking the sensing material and afterwards washing the non-irradiated portion away.

In step c) a second electrode region is prepared on the second side of the raw substrate.

The second electrode region specifically comprises all components which form part of the at least one second electrode of the analyte sensor.

In step c1) a silver composition is applied to the second side of the raw substrate. In an embodiment, the silver composition is applied directly to the second side of the raw substrate. The silver composition may be applied to the second side of the raw substrate so that it covers the second side of the raw substrate at least partially. In another embodiment, the silver composition is applied at least partially to a second conductive trace.

The silver composition may be any composition known to the skilled person. In particular, the silver composition comprises silver. "Silver" within the context of the silver composition of the present invention not only encompasses elemental silver but also silver compounds. In particular, the silver composition comprises Ag/AgCl and a polymer binder. For the polymer binder and for Ag/AgCl the preferences and embodiments described above hold true.

The silver composition may be applied to the second side of the raw substrate by any method known, for example by a wet-coating process. A suitable wet-coating process is for example selected from the group consisting of spin-coating, spray-coating, doctor-blading, printing, dispensing, slot-coating, dip coating and screen printing. After the wet-coating process, the layer of the silver composition may be further treated. Such treatments are for example drying treatments, curing treatments and/or laser ablation treatments. Such treatments are known as such.

In an embodiment, before step c1) a second conductive trace is applied to the raw substrate. For the second conductive trace the embodiments and preferences described above hold true. Thus, the second conductive trace may refer to a second electrically conductive material. For the second conductive material, the embodiments and preferences described above hold true.

The second electrically conductive material can be applied to the first side of the raw substrate by any known method, for example via chemical vapor deposition (CVD), physical vapor deposition (PVD), or a wet-coating process. Wet-coating processes are known as such. A suitable wet-coating process is for example selected from the group consisting of spin-coating, spray-coating, doctor-blading, printing, dispensing, slot-coating, dip coating and screen printing.

In step d) a polymer composition is applied on top of the second electrode region. For the polymer composition the embodiments and preferences described above hold true. A membrane is obtained. The membrane is obtained on the second electrode region. In particular, in step d) a membrane is formed on the second electrode region.

In an embodiment during the inventive process no polymer composition which comprises a hydrophobic polymer is applied to the first side of the substrate.

The polymer composition may be applied on top of the second electrode region by any method known, for example by a wet-coating process. A suitable wet-coating process is for example selected from the group consisting of spin-coating, spray-coating, doctor-blading, printing, dispensing, slot-coating, dip coating and screen printing. After the wet-coating process, the layer of the polymer composition may be further treated. Such treatments are for example drying treatments, curing treatments and/or laser ablation treatments. Such treatments are known as such.

In an embodiment, the polymer composition, after its application to the second side of the raw substrate, preferably the mebrane which is obtained in step d) may be irradiated by a laser to form holes. For the holes, the preferences and embodiments described above for the holes of the membrane hold true.

In step e) the raw substrate comprising the working electrode region, the second electrode region and the membrane is cut to obtain the analyte sensor.

The raw substrate is preferably cut along its width, so that strips are formed. These strips may correspond to the analyte sensor. It is also possible that, before or after the raw substrate is cut along its width that the raw substrate is cut at least once along its length.

The raw substrate is preferably cut with a laser.

Thus, preferably in the method for manufacturing an analyte sensor the cutting in step e) comprises laser-cutting.

When cutting the raw substrate in step e), holes may be formed in the cutting region of the membrane.

Further process steps may be carried out. For example, in a step f) a flux limiting membrane may be applied.

Thus, in an embodiment of the inventive method the following step f) is carried out:

f) applying a flux limiting membrane to the analyte sensor obtained in step e) to obtain a covered analyte sensor.

For the flux limiting membrane, the preferences and embodiments described above hold true. In particular, the flux limiting membrane, preferably the at least one polymeric material comprised in the flux limiting membrane, may for example be applied by a wet-coating process. A suitable wet-coating process is for example selected from the group consisting of spin-coating, spray-coating, doctor-blading, printing, dispensing, slot-coating, dip coating and screen printing.

For example, in a step g) a biocompatibility membrane may be applied.

Thus, in an embodiment of the inventive method, the following step g) is carried out:

g) applying a biocompatibility membrane to the analyte sensor obtained in step e).

If step f) is carried out, then the biocompatibility membrane is typically applied to the covered analyte sensor obtained in step f)

Thus, in case step f) is carried out, in an embodiment the following step g) is carried out:

g) applying a biocompatibility membrane to the covered analyte sensor obtained in step f).

For the biocompatibility membrane, the preferences and embodiments described above hold true.

In particular, the biocompatibility membrane usually consists of a biocompatible material. Thus, preferable, in step g) a biocompatible material is applied. The biocompatibility membrane, preferably the biocompatible material may be applied by any process known, in particular by a wet-coating process. A suitable wet-coating process is for example selected from the group consisting of spin-coating, spray-coating, doctor-blading, printing, dispensing, slot-coating, dip coating and screen printing.

Another object of the present disclosure is, therefore, also an analyte sensor obtainable by the inventive method for manufacturing an analyte sensor.

A further object of the present invention is an analyte sensor system comprising:

- the inventive analyte sensor and
- an electronics unit, the electronics unit being in electronically connected to the analyte sensor.

For the analyte sensor comprised in the analyte sensor system, the embodiments and preferences described above for the inventive analyte sensor hold true.

The term "electronics unit" as used herein is a broad term and is to be given its ordinary and customary meaning to a person of ordinary skill in the art and is not to be limited to a special or customized meaning. The term specifically may refer, without limitation, to a unit, such as a unit which may be handled as a single piece, which is configured for performing at least one electronic function. Specifically, the electronics unit may have at least one interface for being connected to the analyte sensor, wherein the electronics unit may provide at least one electronic function interacting with the analyte sensor, such as at least one measurement function. The electronics unit specifically may be configured for measuring at least one voltage and/or for measuring at least one current, thereby interacting with the analyte sensor. The electronics unit may further comprise at least one integrated circuit, such as a processor and/or a battery. The term "processor" as generally used herein is a broad term and is to be given its ordinary and customary meaning to a person of ordinary skill in the art and is not to be limited to a special or customized meaning. The term specifically may refer, without limitation, to an arbitrary logic circuitry configured for performing basic operations of a computer or system, and/or, generally, to a device which is configured for performing calculations or logic operations. In particular, the processor may be configured for processing an electronic signal, such as a current or a voltage, specifically an electronic signal from the analyte sensor. Specifically, the processor may be or may comprise a microcontroller unit (MCU). Additionally or alternatively, the processor may be or may comprise a microprocessor, thus specifically the processor's elements may be contained in one single integrated circuitry (IC) chip. Additionally or alternatively, the processor may be or may comprise one or more application-specific integrated circuits (ASICs) and/or one or more field-programmable gate arrays (FPGAs) or the like. The processor specifically may be configured, such as by software programming, for performing one or more evaluation operations. Thus, the processor may be configured for processing and/or evaluating the electronic signal from the analyte sensor and, for example, outputting a signal indicating the analyte concentration measured by the analyte sensor. The electronics unit further may comprise at least one measuring device for measuring at least one of a voltage and a current, such as a potentiostat. Further, the electronics unit may comprise a microcontroller, specifically being configured for controlling one or more electronic functions of the electronics unit.

The electronics unit specifically may comprise at least one electronics unit housing, wherein the analyte sensor, e.g. with a proximal end and/or an end providing electrical contacts for contacting the analyte sensor, may protrude into the electronics unit housing and may be electrically connected with at least one electronic component within the electronics unit housing. As an example, the proximal end and/or at least one contact portion of the analyte sensor may protrude into the electronics unit housing and, therein, may be electrically connected to at least one electronic component, such as to at least one printed circuit board and/or at least one contact portion of the electronics unit, e.g. by one or more of a soldering connection, a bonding connection, a plug, a clamping connection or the like. The electronics unit specifically may be used and/or configured as a transmitter for transmitting measurement data to at least one external device, such as to at least one receiver, e.g. wirelessly.

The electronics unit is electronically connected to the analyte sensor. Thus, an electrical connection exits between the analyte sensor and the electronics unit. The electronics unit comprised in the analyte sensor system is in contact with the analyte sensor. For example, the conductive trace and the second conductive trace of the analyte sensor may each form an electrical connection with the electronics unit. Typically, the analyte sensor comprises the contact portion at a proximal end and the working electrode and the second electrode at a distal end. Thus, an electrical signal, such as an electrical current and/or an electric voltage, may be transmitted via the electronic connection from the analyte sensor to the electronics unit. Via the electrical connection, the electronics unit may interact with the analyte sensor for performing at least one electrochemical measurement. The electrical connection specifically, as outlined above, may be established by at least one connection portion of the analyte sensor protruding into a housing of the electronics unit.

**In** the Figures

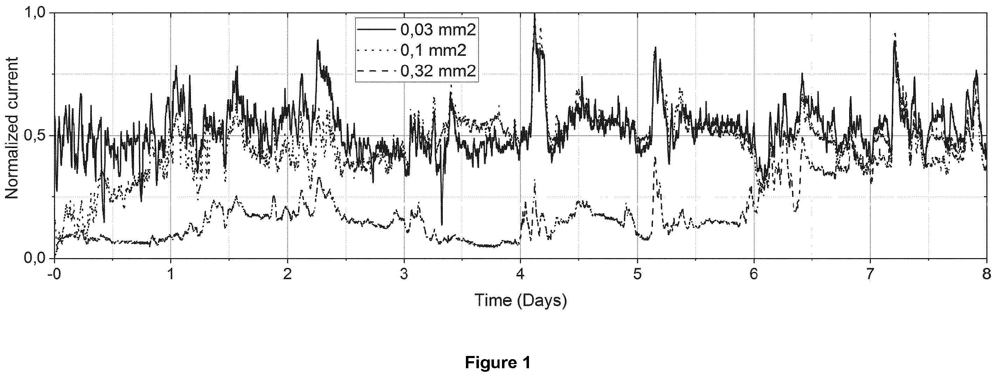

Figure 1:     shows experimental results

Examples

The following examples serve to illustrate the invention. They must not be interpreted as limiting with regard to the scope of protection.

For in vivo tests analyte sensors were prepared, the analyte sensors comprised the following:

- substrate: PET, thickness of 130 μm
- working electrode:

    - electrically conductive material: layer of gold (100 nm thickness) with a layer of carbon paste on top
    - enzyme: glucose oxidase comprised in sensing chemistry (Os-complex modified polymer)

- second electrode: combined counter/reference electrode

    - electrically conductive material: layer of gold (100 nm thickness)
    - silver: Ag/AgCl paste

A membrane comprising a hydrophobic polymer (hydrophobic thermoplastic polyurethane) was located on top of the second electrode. The analyte sensors were laser cut. Holes were formed in the hydrophobic polymer of the analyte sensor depending on the laser cutting conditions of the cutting. A flux limiting membrane and a biocompatibility membrane covered the sensors.

Analyte sensors with the following total area of holes in the hydrophobic polymer were prepared:

| Sensor 1: | 0 mm2 |
|---|---|
| Sensor 2: | 0.03 mm2 |
| Sensor 3: | 0.1 mm2 |
| Sensor 4: | 0.32 mm2 |

The analyte sensors were implanted in vivo in the same subject and the current measured over a period of eight days. In parallel regular blood glucose values (BG values) were measured with a BG meter using finger pricking. Figure 1 shows the normalized current for the measurements with Sensor 2, Sensor 3 and Sensor 4. 0 on the x-axis shows the start of the measurement time after a run-in time of about 1 hour. The measurement curve for Sensor 1 is not shown as no current could be obtained.

Sensor 2 shows from the beginning of the measurement a sufficiently high current which remains constant throughout the whole measurement time (eight days). There are some peaks which indicate high and low glucose values during the measurement time. These peaks correspond to the peaks found in the regular blood glucose value measurements. Thus, the measurement with sensor 2 is particularly reliable and stable even over a longer time period. Further it allows calibration with the regular blood glucose value measurements

Sensor 3 has a significantly longer run-in time, during which the current did not correlate with the BG values. It can be seen from figure 1 that the expected current is only reached after one day of measurement. Thereafter, the measurement is comparable in its reliability and stability with the one of sensor 2.

Sensor 4 exhibited an even longer run-in time, during which the current was significantly lower than expected. Though there are some peaks in the current visible, they did not correlate with the BG values and the sensitivity of the sensor (ratio of the current to the BG values) was too low and instable. After day seven, the current was significantly higher and correlated with the BG values and the sensitivity increased.

**Claims**

1.   An analyte sensor comprising

        - a substrate comprising a first side and a second side;

- at least one working electrode positioned on the first side of the substrate, the at least one working electrode comprising:

   - at least one electrically conductive material and
   - at least one enzyme;

   - at least one second electrode positioned on the second side of the substrate, the at least one second electrode comprising silver;
   - a membrane comprising a polymer composition which comprises a hydrophobic polymer, wherein the hydrophobic polymer has a water uptake of less than 1 % by weight, based on the total weight of the hydrophobic polymer,

   wherein the membrane is located on top of the at least one second electrode, wherein the membrane comprises holes, wherein the total area of holes has a size of at most 0.15 mm$^2$.

2. The analyte sensor according to claim 1, wherein the analyte sensor is an implantable sensor.

3. The analyte sensor according to claim 1 or 2, wherein the at least one second electrode is selected from the group consisting of a counter electrode, a reference electrode and a combined counter/reference electrode.

4. The analyte sensor according to any one of claims 1 to 3, wherein the first side and the second side of the substrate are positioned opposite each other.

5. The analyte sensor according to any one of claims 1 to 4, wherein the at least one second electrode comprises Ag/AgCl.

6. The analyte sensor according to claim 5, wherein the load of AgCl of the at least one second electrode is in the range from 20 μg to 150 μg.

7. The analyte sensor according to any one of claims 1 to 6, wherein the polymer composition comprises a hydrophobic thermoplastic polyurethane.

8. The analyte sensor according to any one of claims 1 to 7, wherein the hydrophobic polymer has a glass transition temperature, wherein the glass transition temperature is in the range from -100 °C to 0 °C.

9. The analyte sensor according to any one of claims 1 to 8, wherein the total area of holes comprised in the membrane is in the range from 0.005 to 0.15 mm$^2$, preferably in the range from 0.005 to 0.05 mm$^2$.

10. The analyte sensor according to any one of claims 1 to 9, wherein the at least one working electrode is free of the membrane comprising the polymer composition which comprises the hydrophobic polymer.

11. A method for manufacturing an analyte sensor according to any one of claims 1 to 10, the method comprising the steps:

   a) providing a raw substrate which comprises a first side and a second side,
   b) preparing a working electrode region on the first side of the raw substrate, the preparing of the working electrode region comprising the steps:

      b1) applying an electrically conductive material to the first side of the raw substrate,
      b2) applying a sensing material comprising at least one enzyme at least partially on the electrically conductive material,

   c) preparing a second electrode region on the second side of the raw substrate, the preparing of the second electrode region comprising the steps:
   c1) applying a silver composition on the second side of the raw substrate,
   d) applying a polymer composition on top of the second electrode region, to obtain a membrane, wherein the polymer composition comprises a hydrophobic polymer, wherein the hydrophobic polymer has a water uptake of less than 1 % by weight, based on the total weight of the hydrophobic polymer,
   e) cutting the raw substrate comprising the working electrode region, the second electrode region and the membrane to obtain the analyte sensor.

12. The method according to claim 11, wherein the cutting in step e) comprises laser-cutting.

13. An analyte sensor system comprising

   - an analyte sensor according to any one of claims 1 to 10,
   - an electronics unit, the electronics unit being configured to electronically connect to the analyte sensor.

**Patentansprüche**

1. Analytsensor, umfassend

   - ein Substrat, das eine erste Seite und eine zweite Seite umfasst;
   - mindestens eine Arbeitselektrode, die auf der ersten Seite des Substrats positioniert ist, wobei die mindestens eine Arbeitselektrode Folgendes umfasst:

- mindestens ein elektrisch leitfähiges Material und
- mindestens ein Enzym;

- mindestens eine zweite Elektrode, die auf der zweiten Seite des Substrats positioniert ist, wobei die mindestens eine zweite Elektrode Silber umfasst;
- eine Membran, die eine Polymerzusammensetzung umfasst, die ein hydrophobes Polymer umfasst, wobei das hydrophobe Polymer eine Wasseraufnahme von weniger als 1 Gew.-%, bezogen auf das Gesamtgewicht des hydrophoben Polymers, aufweist,

wobei sich die Membran auf der mindestens einen zweiten Elektrode befindet, wobei die Membran Löcher umfasst, wobei die Gesamtfläche der Löcher eine Größe von höchstens 0,15 mm$^2$ aufweist.

2. Analytsensor nach Anspruch 1, wobei der Analytsensor ein implantierbarer Sensor ist.

3. Analytsensor nach Anspruch 1 oder 2, wobei die mindestens eine zweite Elektrode ausgewählt ist aus der Gruppe bestehend aus einer Gegenelektrode, einer Referenzelektrode und einer kombinierten Gegen-/Referenzelektrode.

4. Analytsensor nach einem der Ansprüche 1 bis 3, wobei die erste Seite und die zweite Seite des Substrats einander gegenüberliegend positioniert sind.

5. Analytsensor nach einem der Ansprüche 1 bis 4, wobei die mindestens eine zweite Elektrode Ag/AgCl umfasst.

6. Analytsensor nach Anspruch 5, wobei die AgCl-Beladung der mindestens einen zweiten Elektrode im Bereich von 20 μg bis 150 μg liegt.

7. Analytsensor nach einem der Ansprüche 1 bis 6, wobei die Polymerzusammensetzung ein hydrophobes thermoplastisches Polyurethan umfasst.

8. Analytsensor nach einem der Ansprüche 1 bis 7, wobei das hydrophobe Polymer eine Glasübergangstemperatur aufweist, wobei die Glasübergangstemperatur im Bereich von -100 °C bis 0 °C liegt.

9. Analytsensor nach einem der Ansprüche 1 bis 8, wobei die Gesamtfläche der Löcher, die in der Membran enthalten sind, im Bereich von 0,005 bis 0,15 mm$^2$, vorzugsweise im Bereich von 0,005 bis 0,05 mm$^2$, liegt.

10. Analytsensor nach einem der Ansprüche 1 bis 9, wobei die mindestens eine Arbeitselektrode frei von der Membran ist, die die Polymerzusammensetzung umfasst, welche das hydrophobe Polymer umfasst.

11. Verfahren zur Herstellung eines Analytsensors nach einem der Ansprüche 1 bis 10, wobei das Verfahren die folgenden Schritte umfasst:

a) Bereitstellen eines Rohsubstrats, das eine erste Seite und eine zweite Seite umfasst,
b) Herstellen eines Arbeitselektrodenbereichs auf der ersten Seite des Rohsubstrats, wobei das Herstellen des Arbeitselektrodenbereichs die folgenden Schritte umfasst:

b1) Aufbringen eines elektrisch leitfähigen Materials auf die erste Seite des Rohsubstrats,
b2) Aufbringen eines Sensormaterials, das mindestens ein Enzym umfasst, mindestens teilweise auf das elektrisch leitfähige Material,

c) Herstellen eines zweiten Elektrodenbereichs auf der zweiten Seite des Rohsubstrats, wobei das Herstellen des zweiten Elektrodenbereichs die folgenden Schritte umfasst:
c1) Aufbringen einer Silberzusammensetzung auf die zweite Seite des Rohsubstrats,
d) Aufbringen einer Polymerzusammensetzung auf den zweiten Elektrodenbereich, sodass eine Membran erhalten wird, wobei die Polymerzusammensetzung ein hydrophobes Polymer umfasst, wobei das hydrophobe Polymer eine Wasseraufnahme von weniger als 1 Gew.-% aufweist, bezogen auf das Gesamtgewicht des hydrophoben Polymers,
e) Schneiden des Rohsubstrats, das den Arbeitselektrodenbereich, den zweiten Elektrodenbereich und die Membran umfasst, um den Analytsensor zu erhalten.

12. Verfahren nach Anspruch 11, wobei das Schneiden in Schritt e) Laserschneiden umfasst.

13. Analytsensorsystem, umfassend

- einen Analytsensor nach einem der Ansprüche 1 bis 10,
- eine Elektronikeinheit, wobei die Elektronikeinheit konfiguriert ist, um mit dem Analytsensor elektronisch verbunden zu werden.

**Revendications**

1. Capteur d'analytes comprenant

   - un substrat comprenant un premier côté et un second côté ;
   - au moins une électrode de travail positionnée sur le premier côté du substrat, l'au moins une électrode de travail comprenant :

      - au moins un matériau électriquement conducteur et
      - au moins une enzyme ;

   - au moins une seconde électrode positionnée sur le second côté du substrat, l'au moins une seconde électrode comprenant de l'argent ;
   - une membrane comprenant une composition polymère qui comprend un polymère hydrophobe, le polymère hydrophobe ayant une absorption d'eau de moins de 1 % en poids, en se basant sur le poids total du polymère hydrophobe,

   dans lequel la membrane est située au sommet de l'au moins une seconde électrode, la membrane comprenant des orifices, l'aire totale des orifices étant d'au moins 0,15 mm².

2. Capteur d'analytes selon la revendication 1, dans lequel le capteur d'analytes est un capteur implantable.

3. Capteur d'analytes selon la revendication 1 ou 2, dans lequel l'au moins une seconde électrode est choisie dans le groupe constitué par une contre-électrode, une électrode de référence et une contre-électrode/une électrode de référence combinées.

4. Capteur d'analytes selon l'une quelconque des revendications 1 à 3, dans lequel le premier côté et le second côté du substrat sont positionnés de manière opposée l'un à l'autre.

5. Capteur d'analytes selon l'une quelconque des revendications 1 à 4, dans lequel l'au moins une seconde électrode comprend de l'Ag/AgCl.

6. Capteur d'analytes selon la revendication 5, dans lequel la charge d'AgCl de l'au moins une seconde électrode est dans la plage de 20 μg à 150 μg.

7. Capteur d'analytes selon l'une quelconque des revendications 1 à 6, dans lequel la composition polymère comprend un polyuréthane thermoplastique hydrophobe.

8. Capteur d'analytes selon l'une quelconque des revendications 1 à 7, dans lequel le polymère hydrophobe a une température de transition vitreuse, dans lequel la température de transition vitreuse est dans la plage de -100 °C à 0 °C.

9. Capteur d'analytes selon l'une quelconque des revendications 1 à 8, dans lequel l'aire totale des orifices compris dans la membrane est dans la plage de 0,005 à 0,15 mm², de préférence dans la plage de 0,005 à 0,05 mm².

10. Capteur d'analytes selon l'une quelconque des revendications 1 à 9, dans lequel l'au moins une électrode de travail est exempte de la membrane comprenant la composition polymère qui comprend le polymère hydrophobe.

11. Procédé de fabrication d'un capteur d'analytes selon l'une quelconque des revendications 1 à 10, le procédé comprenant les étapes :

    a) fourniture d'un substrat brut qui comprend un premier côté et un second côté,
    b) préparation d'une région d'électrode de travail sur le premier côté du substrat brut, la préparation de la région d'électrode de travail comprenant les étapes :

       b1) application d'un matériau électriquement conducteur au premier côté du substrat brut,
       b2) application d'un matériau de détection comprenant au moins une enzyme au moins partiellement sur le matériau électriquement conducteur,

    c) préparation d'une seconde région d'électrode sur le second côté du substrat brut, la préparation de la seconde région d'électrode comprenant les étapes :
    c1) application d'une composition d'argent sur le second côté du substrat brut,
    d) application d'une composition polymère au sommet de la seconde région d'électrode, pour obtenir une membrane, la composition polymère comprenant un polymère hydrophobe, le polymère hydrophobe ayant une absorption d'eau de moins de 1 % en poids, en se basant sur le poids total du polymère hydrophobe,
    e) découpe du substrat brut comprenant la région d'électrode de travail, la seconde région d'électrode et la membrane pour obtenir le capteur d'analytes.

12. Procédé selon la revendication 11, dans lequel la découpe de l'étape e) comprend une découpe laser.

**13.** Système de capteur d'analytes comprenant

- un capteur d'analytes selon l'une quelconque des revendications 1 à 10,
- une unité électronique, l'unité électronique étant configurée pour être connectée électroniquement au capteur d'analytes.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9895091 B2
- US 10470691 B2
- WO 0136660 A2
- EP 2697388 A1
- WO 2007071562 A1
- WO 2005078424 A1
- WO 2019166394 A1

**Non-patent literature cited in the description**

- **FELDMANN et al.** *Diabetes Technology & Therapeutics*, 2003, vol. 5 (5), 769-779